# EUROPEAN PATENT APPLICATION

(11) **EP 4 825 381 A1**
(43) Date of publication of application: **21.10.2026**
(21) Application number: 25195785.8
(22) Date of filing: 13.08.2025
(51) Int. Cl.: A61K 31/554, A61K 9/00, A61P 31/14

(54) **USE OF ZIRESOVIR IN THE PREVENTION OR TREATMENT OF SEQUELAE OF HUMAN RESPIRATORY SYNCYTIAL VIRUS INFECTION**

(30) Priority: 14.04.2025 CN 202510467023
(71) Applicant: Shanghai Ark Biopharmaceutical Co. Ltd., Pudong New Area Shanghai 201203 (CN)
(72) Inventor: WU, Jim Zhen, Shanghai, 201203 (CN); ZHU, Xiaoping, Shanghai, 201203 (CN)
(74) Representative: Pfenning, Meinig & Partner mbB

(57) **Abstract**

Provided in present disclosure is a new use of ziresovir, and specifically the use of ziresovir in preventing or treating sequelae of human respiratory syncytial virus infection.

## Description

This application claims priority to Chinese Patent Application No.202510467023.1, filed on April 14, 2025, which is incorporated by reference for all purposes as if fully set forth herein.

### TECHNICAL FIELD

The present disclosure relates to the technical field of antiviral infection, and pertains to a new use of ziresovir. Specifically, it relates to the use of ziresovir in the preparation of drugs for preventing or treating the sequelae of human respiratory syncytial virus infection.

### BACKGROUND

Human respiratory syncytial virus (RSV) is an enveloped single-stranded RNA virus. It is a highly contagious pathogen and a common cause of acute lower respiratory tract infections in adults. The prevalence of respiratory syncytial virus usually exhibits a distinct seasonal pattern and there are geographical gradient differences. Its two major strains, namely RSV-A and RSV-B (both having multiple genotypes), can circulate simultaneously or alternately. This virus is easily transmitted through close contact with aerosolized droplets or contaminated objects, as well as through contact with the nasal mucosa or conjunctiva of the eye. The incubation period after infection is 2 to 8 days. Initially, the infection is confined to nasopharyngeal tissues, but it may progress to bronchiolitis and pneumonia. The immunity that the human body, especially infants and young children, develops against respiratory syncytial virus does not provide long-term protection. Therefore, repeated infections are quite common throughout a person's life. Healthy adults usually only experience mild symptoms after infection, but severe complications may occur in high-risk groups, such as children under 5 years old, individuals aged great than or equal to 75 years, those with weakened immune systems and/or severe pulmonary/cardiovascular comorbidities. It is estimated that globally in 2019, respiratory syncytial virus led to approximately 33 million cases of lower respiratory tract infections, 3.6 million hospitalizations, and over 100,000 deaths among children under 5 years old. Nearly 70% of these deaths occurred outside of medical institutions.

Despite decades of great efforts in developing antiviral drugs against respiratory syncytial virus , the main treatment for acute RSV infection remains supportive care, including fluid replacement, oxygen supplementation, airway suction, and mechanical ventilation when necessary (American Academy of Pediatrics. Respiratory syncytial virus. In: Kimberlin DW, Brady MT, Jackson MA, editors. Red Book: 2018-2021 Report of the Committee on Infectious Diseases. Elk Grove Village: American Academy of Pediatrics; 2018. p. 682-92). Ribavirin is the only approved antiviral therapy for RSV infection. However, due to concerns about its genotoxicity and the safety of healthcare workers, as well as the inconvenience of administration route (aerosol), it is currently used to treat only life-threatening RSV infections in immuno-compromised patients, and only approved in an aerosolized formulation for pediatric patients. Therefore, RSV prophylaxis has become an crucial means of preventing severe RSV infections in high-risk infants and young children. Between 2022 and 2023, an RSV maternal vaccine and a long-acting monoclonal antibody (mAb) for the prevention of lower respiratory tract infections (LRTIs) caused by RSV were approved for use in all infants born during or approaching their first RSV epidemic season, as well as in high-risk children under 2 years of age. Studies have shown that these measures are highly effective and practical in preventing severe LRTIs caused by RSV, and they have been quickly adopted in many high-income countries. However, in low- and middle-income countries (LMICs), their accessibility remains very limited.

Early-life RSV LRTI has also been associated with subsequent long-term respiratory sequelae, including recurrent LRTI, recurrent wheezing, asthma, and lung function impairment. These sequelae may persist into adulthood in the form of chronic respiratory diseases. There is a well-established association between RSV lower respiratory tract infection and subsequent long-term risk of recurrence of wheeze in early childhood and asthma, as reported in a population-based, prospective birth-cohort study (Rosas-Salazar C, Chirkova T, Gebretsadik T, et al. Respiratory syncytial virus infection during infancy and asthma during childhood in the USA (INSPIRE): a population-based, prospective birth cohort study. Lancet 2023; 401: 1669-80) and a systematic review of 74 studies (Fauroux B, Simoes EAF, Checchia PA, et al. The burden and long-term respiratory morbidity associated with respiratory syncytial virus infection in early childhood. Infect Dis Ther 2017; 6: 173-97). Some studies have suggested that greater disease severity and viral load increase the risks of recurrence of wheeze in early childhood and asthma, whereas others have not found such an association. Whether this is a causal association or a possible shared allergic disposition has not convincingly been established (Driscoll AJ, Arshad SH, Bont L, et al. Does respiratory syncytial virus lower respiratory illness in early life cause recurrent wheeze of early childhood and asthma? Critical review of the evidence and guidance for future studies from a World Health Organization sponsored meeting. Vaccine 2020; 38: 2435-48; Shi T, Ooi Y, Zaw EM, et al. Association between respiratory syncytial virus-associated acute lower respiratory infection in early life and recurrent wheeze and asthma in later childhood. J Infect Dis 2020; 222 (suppl 7): S628-33). There is evidence that RSV infection in early life decreases T-cell function and might increase propensity to allergic airway disease; the role such immunological changes could play in the etiology of recurrence of wheeze in early childhood is unclear (Shi T, Ooi Y, Zaw EM, et al. Association between respiratory syncytial virus-associated acute lower respiratory infection in early life and recurrent wheeze and asthma in later childhood. J Infect Dis 2020; 222 (suppl 7): S628-33; Krishnamoorthy N, Khare A, Oriss TB, et al. Early infection with respiratory syncytial virus impairs regulatory T cell function and increases susceptibility to allergic asthma. Nat Med 2012; 18: 1525-30).

Existing studies have shown that vaccination or antibody injection can effectively prevent LTRI caused by RSV in high-risk children, but their long-term effects on the development of asthma and recurrent wheezing remain unclear and need to be confirmed through further research. For example, some studies have shown that palivizumab prophylaxis against RSV infection has no effect on recurrent wheezing or asthma (Palivizumab prophylaxis in preterm infants and subsequent wheezing/asthma: 10-year follow-up study, Pediatr Pulmonol. 2024 Mar;59(3):743-749). Also, some studies have shown that palivizumab prophylaxis in preterm infants leads to a significantly reduced incidence of recurrent wheezing in the first six years (Palivizumab Prophylaxis in Preterm Infants and Subsequent Recurrent Wheezing. Six-Year Follow-up Study, Am J Respir Crit Care Med. 2017 Jul 1;196(1):29-38).

Although ribavirin as the only approved drug for the treatment of acute RSV infection, can inhibit the replication of RSV during the active replication stage through multiple mechanisms, it has found no significant long-term benefits in reducing wheezing, asthma, or other respiratory sequelae after RSV infection according to multiple clinical studies and meta-analyses. Krilov and coworkers reported the study to determine whether therapy for RSV LRTI with ribavirin would reduce long-term sequelae. Thirty-three children who had received ribavirin for RSV LRTI in the 1986/1987 season and 67 age-matched control children who had not received ribavirin were monitored for 5 to 6 yr. Those in the ribavirin group had more severe diseases at presentation, but there was no between-group difference in long-term outcomes with respect to reactive airway disease (RAD) or pulmonary function (Krilov LR, Mandel FS, Barone SR, Fagin JC, Bronchiolitis Study Group. Follow-up of children with respiratory syncytial virus bronchiolitis in 1986 and 1987: potential effect of ribavirin on long term pulmonary function. Pediatr Infect Dis J,1997;16:273-276). Long and associates (11) enrolled 54 children who had participated in randomized trials of ribavirin therapy into a 9-year follow-up study to assess the long-term effects of the antiviral agent. In this study, 28 children had been treated with ribavirin and 26 had been treated with placebo. There were no between-group differences with respect to any of the parameters evaluated, including recurrent LRTI, wheezing, and pulmonary function, suggesting no benefit of ribavirin therapy (Long CE, Voter KZ, Barker WH, Hall CB. Long term follow-up of children hospitalized with respiratory syncytial virus lower respiratory tract infection and randomly treated with ribavirin or placebo. Pediatr Infect Dis J 1997;16:1023-1028). Rodriguez and coworkers (Rodriguez WJ, Arrobio J, Fink R, Kim HW, Milburn C. Prospective follow- up and pulmonary functions from a placebo-controlled randomized trial of ribavirin therapy in respiratory syncytial virus bronchiolitis. Ribavirin Study Group. Arch Pediatr Adolesc Med 1999;153:469-474) conducted a study with long-term follow-up in 24 ribavirin recipients and 11 placebo control subjects, 13 and 6 of whom, respectively, underwent pulmonary function testing (PFT). The investigators reported minimal improvement in respiratory outcome. There was no difference in reactive airway disease, wheezing, or pneumonia between the two groups for up to 7 yr. There was no between-group difference in results of methacholine challenge tests, but 0 of 6 placebo recipients had normal or mildly abnormal PFTs, compared with 6 of 13 ribavirin recipients. Thus, these long-term follow-up studies showed no real difference in long-term outcomes with antiviral therapy.

Currently, several compounds have been proposed as inhibitors of RSV and have entered Phase I or Phase II stage, such as EDP-938, sisunatovir, ASC10, VV116, etc. However, these investigational antiviral drugs only focus on viral clearance and symptom relief in the acute phase of infection, and there lacks a solution for long-term adverse consequences.

Therefore, there is a need to provide methods for treating or preventing the sequelae of respiratory syncytial virus infection.

In view of this, the present disclosure is specifically proposed.

### SUMMARY

Ziresovir (CAS No. 1422500-60-4), also known as RO-0529, AK0529, is a fusion protein inhibitor of respiratory syncytial virus, and the structural formula thereof is as follows:

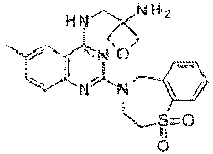

Provided in present disclosure is the use of ziresovir or a pharmaceutically acceptable salt thereof in significantly reducing the sequelae of RSV infection.

### Problems to be Solved by Present Invention

The first objective of the present disclosure is to provide the use of ziresovir or its pharmaceutically acceptable salt in the preparation of a drug for preventing or treating the sequelae of human respiratory syncytial virus (RSV) infection.

Preferably, the population infected with human respiratory syncytial virus is infants, children, or adults.

Preferably, the population infected with human respiratory syncytial virus is infants aged 1 to 24 months.

Preferably, the population infected with human respiratory syncytial virus is infants aged 1 to 6 months.

Preferably, the sequelae of human respiratory syncytial virus infection are selected from the group consisting of cough, wheezing, asthma, shortness of breath, reactive airway disease, recurrent respiratory tract infections, and impaired lung function.

Preferably, the sequelae of human respiratory syncytial virus infection are selected from the group consisting of wheezing and/or asthma.

Preferably, the use reduces at least one sequela selected from the group consisting of the annual occurrence of wheeze, number of wheeze episodes, and risk of developing asthma.

Preferably, the use reduces the annualised rate of wheeze by a proportion of at least 30%, preferably at least 50%, and more preferably at least 70%.

Preferably, the use reduces the incidence of wheeze episodes by a proportion of at least 30%, preferably at least 40%, and more preferably at least 60%.

Preferably, the use reduces the prevalence rate of asthma by a proportion of at least 30%, preferably at least 40%, and more preferably at least 50%.

Preferably, the dosage of ziresovir or its pharmaceutically acceptable salt administrated to a subject is approximately 1 mg/kg/d to 20 mg/kg/d, preferably 2 mg/kg/d to 15 mg/kg/d, and more preferably 4 mg/kg/d to 12 mg/kg/d.

Preferably, the ziresovir or its pharmaceutically acceptable salt is administrated twice a day.

Preferably, the ziresovir or its pharmaceutically acceptable salt is administered orally for 5 consecutive days.

Preferably, the ziresovir or its pharmaceutically acceptable salt is administered during the treatment period of the acute infection.

The second objective of the present disclosure is to provide a use of the pharmaceutical composition in the preparation of a drug for preventing or treating sequelae of human respiratory syncytial virus infection, the pharmaceutical composition comprising ziresovir and at least one pharmaceutically acceptable ingredient.

Preferably, the ingredients include one or more selected from the group consisting of fillers, excipients, stabilizers, diluents, binders, disintegrants, lubricants, glidants, wetting agents, effervescent agents, colorants, sweeteners, flavoring agents, preservatives, dispersants, film-forming agents, plasticizers, pore-forming agents, opacifiers, retardants, and solvents.

Preferably, the drug or pharmaceutical composition is in the form of powder, pill, tablet, capsule, oral liquid, aerosol or injection, preferably capsule.

The third objective of the present disclosure is to provide a method for preventing or treating sequelae of human respiratory syncytial virus infection, comprising administering an effective amount of ziresovir or its pharmaceutically acceptable salt, to a subject in need thereof, where the effective amount is 1~20 mg/kg/d, preferably 2~15 mg/kg/d, more preferably 4~12 mg/kg/d.

Preferably, the ziresovir is administered during the treatment period of the acute infection.

Preferably, the effective amount for ziresovir or its pharmaceutically acceptable salt is administered twice a day.

Preferably, the effective amount for ziresovir or its pharmaceutically acceptable salt is administered orally for 5 consecutive days.

Preferably, the population having the human respiratory syncytial virus infection is infant, child, or adult.

Preferably, the population having the human respiratory syncytial virus infection is infant aged 1 to 24 months, preferably infant aged 1 to 6 months.

Preferably, the sequelae of human respiratory syncytial virus infection are selected from the group consisting of cough, wheezing, asthma, shortness of breath, reactive airway disease, recurrent respiratory tract infections, and pulmonary function impairments.

Preferably, the sequelae of human respiratory syncytial virus infection are selected from the group consisting of wheezing and/or asthma.

Preferably, the method reduces at least one sequela selected from the group consisting of annual occurrence of wheezing, number of wheeze episodes, and probability of developing asthma.

Preferably, the method reduces the annualised rate of wheeze by a proportion of at least 30%, preferably at least 50%, and more preferably at least 70%.

Preferably, the method reduces the incidence of wheeze episodes by a proportion of at least 30%, preferably at least 40%, and more preferably at least 60%.

Preferably, the method reduces the prevalence rate of asthma by a proportion of at least 30%, preferably at least 40%, and more preferably at least 50%.

### Effects of the Disclosure:

Confirmed in present disclosure is that ziresovir is a promising therapeutic drug for treating RSV-related bronchiolitis, and particularly demonstrates good efficacy and tolerability in patients aged 6 months or younger. In addition to the significantly relieved symptoms and the reduced viral load during the acute treatment period, ziresovir can also provide long-term benefits, such as the decreased post-infection incidence of wheezing, which potentially provides significant benefits in children's respiratory health.

### BRIEF DESCRIPTION OF THE DRAWINGS

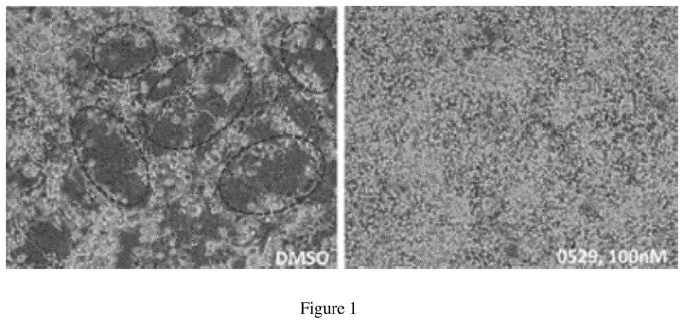

Figure 1 shows AK0529 inhibiting RSV F protein-induced cell-cell fusion.

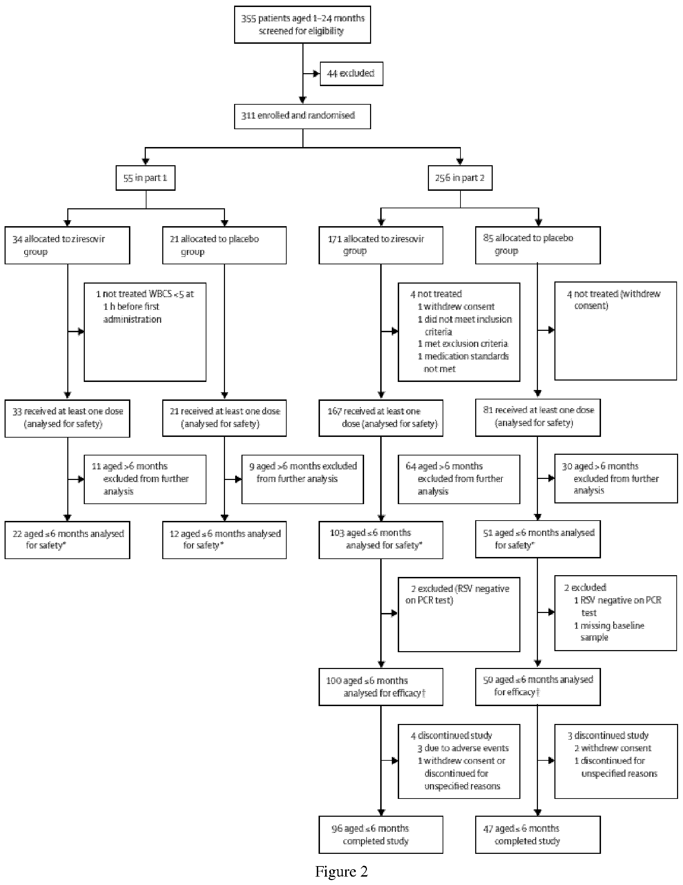

Figure 2 shows the designed trial profile of the present disclosure, where* represents safety analysis set of patients aged 6 months or younger reported in this study (n=188), and †represents intention-to-treat infected analysis set of patients aged 6 months or younger reported in this study (n=150).

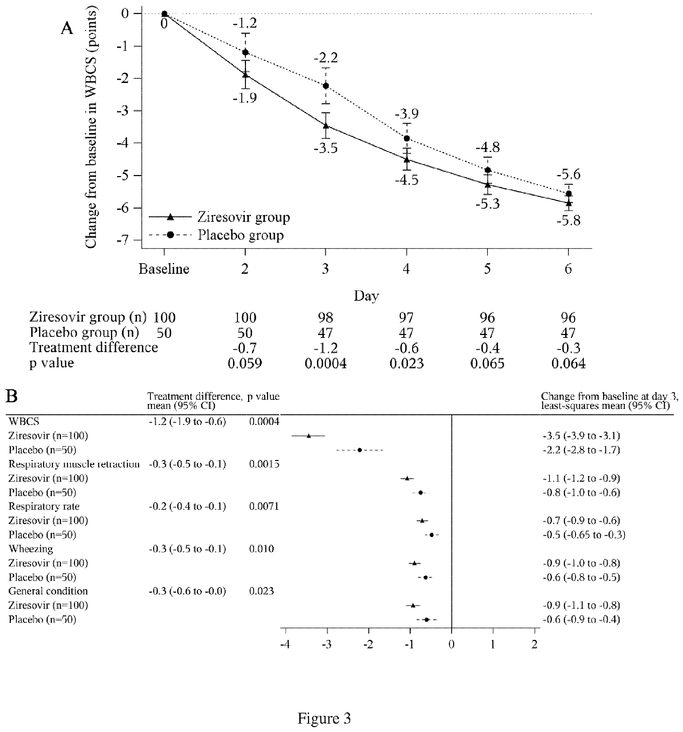

Figure 3 shows change in WBCS (primary endpoint) and component scores among patients aged 6 months or younger, where (A) represents change from baseline in WBCS over time; and (B) represents change from baseline in WBCS and component scores on day 3. All graphs show least-squares mean values, with error bars representing 95% CIs. Treatment difference shows the between-group difference in change from baseline in WBCS and component sores, with negative values indicating a greater decrease in the ziresovir group than the placebo group. RSV=respiratory syncytial virus. WBCS=Wang bronchiolitis clinical score.

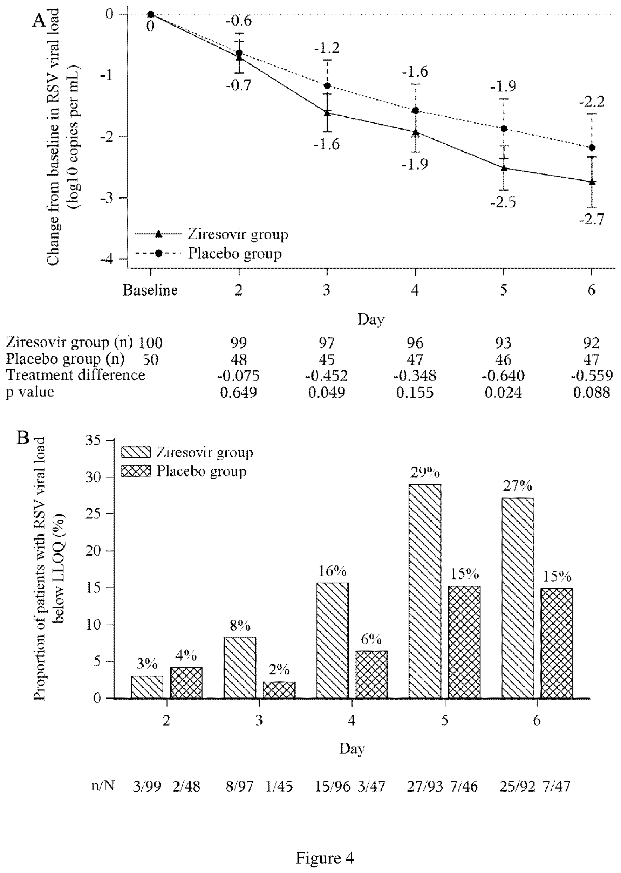

Figure 4 shows RSV viral load endpoints in patients aged 6 months or younger, where (A) represents change from baseline in RSV viral load. Graph shows least-squares mean values, with error bars representing 95% CIs. Treatment difference shows the between-group difference in change from baseline in RSV viral load, with negative values indicating a greater decrease in the ziresovir group than the placebo group. (B) represents proportions of patients with a viral load under the LLOQ. LLOQ=lower limit of quantification. RSV=respiratory syncytial virus.

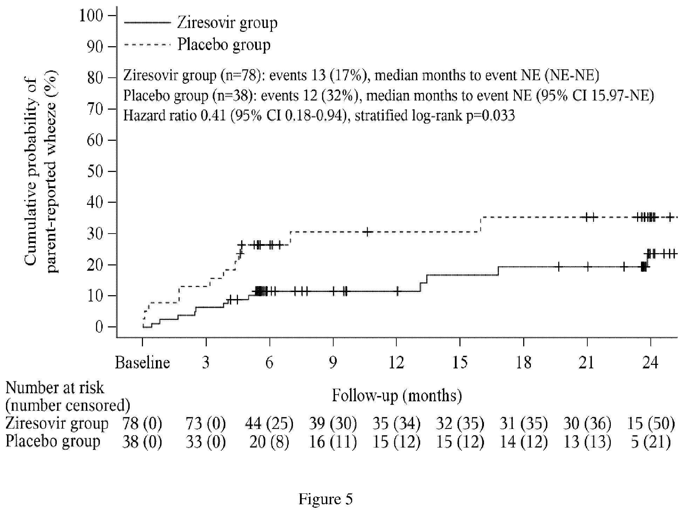

Figure 5 shows Kaplan-Meier curve of time to first parent-reported wheeze during follow-up of patients aged 6 months or younger. Patients reported as censored include those who did not have any reported wheezing episodes during the 2-year follow-up period, or who discontinued or were otherwise unavailable for complete follow-up. NE=not evaluable.

### DETAILED DESCRIPTION

To make the technical solutions and beneficial effects of present disclosure more obvious and easier to understand, the following description is explained in detail by enumerating specific embodiments. The drawings are not necessarily drawn to scale, and local features can be enlarged or narrowed to show the details of local features more clearly. Unless otherwise defined, the technical and scientific terms used in this text have the same meanings as those in the technical field to which present disclosure belongs.

Respiratory syncytial virus (RSV) has a greater impact on children aged 2 years or younger, especially infants aged 6 months or younger. It can easily lead to lower respiratory tract infections, such as wheezing pneumonia, and respiratory failure and breathing difficulties in severe cases. RSV infection not only brings about immediate effects but may also cause long-term sequelae, such as wheezing and asthma, which have a lifelong impact on children's lung function.

The inventors of present disclosure have found through researches that ziresovir has a good therapeutic effect on RSV-associated bronchiolitis, especially in infants of young months of age; ziresovir can remarkably alleviate the symptoms of RSV infection, reduce the viral load, and improve the clinical symptoms; ziresovir, more importantly, can also significantly reduce the sequelae of RSV infection, particularly the incidence of parents-reported wheezing that potentially offers important benefits for children's respiratory health. The present disclosure is proposed based on this.

The first object of present disclosure is to provide the use of ziresovir or a pharmaceutically acceptable salt thereof in reducing the sequelae of RSV infection.

In present disclosure, ziresovir was produced from commercially available conventional raw materials and also be synthesized by known and publicly disclosed methods. Ziresovir, its preparation, crystal structure and manufacturing process are described in the international publications WO2013020993A1, WO2017009316A1 and WO2015110446A1. The content of each of the above-mentioned documents is incorporated herein by reference in its entirety.

In preferred embodiments, diseases caused by human respiratory syncytial virus (RSV) infection include bronchiolitis caused by human RSV and pneumonia caused by human RSV.

In preferred embodiments, provided is the use of ziresovir in the preparation of a drug for reducing or alleviating the sequelae of human respiratory syncytial virus infection.

In preferred embodiments, the population having the human respiratory syncytial virus infection is infant, child, or adult.

In preferred embodiments, the population having the human respiratory syncytial virus infection is infant aged 1 to 24 months.

In preferred embodiments, the population having the human respiratory syncytial virus infection is infant aged 1 to 6 months.

In preferred embodiments, the sequelae of human respiratory syncytial virus infection are selected from the group consisting of cough, wheezing, asthma, shortness of breath, reactive airway disease, recurrent respiratory tract infections, and pulmonary function impairments

In preferred embodiments, the sequelae of human respiratory syncytial virus infection are selected from the group consisting of wheezing and/or asthma.

The pharmaceutical or pharmaceutical composition of the present disclosure can be administered intravenously, orally, rectally or sublingually. In another embodiment, the route of administration is oral. In one embodiment, the route of administration is intravenous injection. In another embodiment, the route of administration is rectal administration. In yet another embodiment, the route of administration is sublingual.

In the present disclosure, the dosage of ziresovir received by the subject is based on the subject's body weight. In preferred embodiments, the dosage of ziresovir is approximately 1 to 20 mg/kg/d, for example, 1 mg/kg/d, 2 mg/kg/d, 3 mg/kg/d, 4 mg/kg/d, 5 mg/kg/d, 6 mg/kg/d, 7 mg/kg/d, 8 mg/kg/d, 9 mg/kg/d, 10 mg/kg/d, 11 mg/kg/d, 12 mg/kg/d, 13 mg/kg/d, 14 mg/kg/d, 15 mg/kg/d, 16 mg/kg/d, 17 mg/kg/d, 18 mg/kg/d, 19 mg/kg/d, 20 mg/kg/d. In preferred embodiments, the dosage of ziresovir is approximately 2 to 15 mg/kg/d.

In preferred embodiments, the dosage of ziresovir is approximately 4 to 12 mg/kg/d.

In preferred embodiments, the drug is administered 1, 2, 3, or 4 times a day, preferably twice a day.

In preferred embodiments, the administration period of the drug is 1 to 10 days or longer, preferably 1 day, 3 days, 5 days, 7 days, 9 days, or 10 days. Optionally, the duration of each administration period is identical or different, and the intervals between each administration period are identical or different.

In preferred embodiments, the use reduces the annualised rate of wheeze by a proportion of at least 30%, preferably at least 50%, and more preferably at least 70%. For example, the use reduces the annualised rate of wheeze by a proportion of 30% to 80%, 35% to 80%, 40% to 80%, 50% to 80%, 60% to 80%, 70% to 80%, 30% to 75%, 35% to 75%, 40% to 75%, 45% to 75%, 50% to 75%, 30%, 35%, 40%, 45%, 50%, 55%, 60%, 65%, 70%, 75%, or 72%.

In preferred embodiments, the use reduces the incidence of wheeze episodes by a proportion of at least 30%, preferably at least 40%, and more preferably at least 60%. For example, the use reduces the incidence of wheeze episodes by a proportion of 30% - 80%, 35% - 80%, 40% - 80%, 50% - 80%, 60% - 80%, 30% - 70%, 35% - 70%, 40% - 70%, 50% - 70%, 60% - 70%, 30% - 65%, 35% - 65%, 40% - 65%, 50% - 65%, 55% - 65%, 30%, 35%, 40%, 45%, 50%, 55%, 60%, 61.5%.

In preferred embodiments, the use reduces the prevalence rate of asthma by a proportion of at least 30%, preferably at least 40%, and more preferably at least 50%. For example, the use reduces the prevalence rate of asthma by a proportion of 30% - 60%, 35% - 60%, 40% - 60%, 50% - 60%, 30% - 55%, 35% - 55%, 40% - 55%, 45% - 55%, 50% - 55%, 30%, 35%, 40%, 45%, 50%, 51.3%.

The second objective of present disclosure is to provide the use of a pharmaceutical composition in the preparation of a drug for preventing or treating the sequelae of human respiratory syncytial virus infection, wherein the pharmaceutical composition comprises ziresovir and at least one pharmaceutically acceptable ingredient.

In preferred embodiments, the ingredients include one or more of fillers, excipients, stabilizers, diluents, binders, disintegrants, lubricants, glidants, wetting agents, effervescent agents, colorants, sweeteners, flavoring agents, preservatives, dispersants, film-forming agents, plasticizers, pore-forming agents, opacifiers, retardants, and solvents.

In preferred embodiments, the dosage form of the drug or pharmaceutical composition is powder, pill, tablet, capsule, oral liquid, aerosol or injection.

In preferred embodiments, the dosage form of the drug or pharmaceutical composition is capsule.

The third object of the present disclosure is to provide a method for preventing or treating the sequelae of human respiratory syncytial virus infection, comprising administering an effective dose of ziresovir or its pharmaceutically acceptable salt to subject in need thereof, wherein the effective dose is 1 to 20 mg/kg/d, preferably 2 to 15 mg/kg/d, and more preferably 4 to 12 mg/kg/d.

In preferred embodiments, the ziresovir or its pharmaceutically acceptable salt is administered during the treatment period of the acute infection.

In preferred embodiments, the ziresovir or its pharmaceutically acceptable salt is administered twice a day. For example, the effective dose is administered orally for 5 consecutive days.

In preferred embodiments, the population having the human respiratory syncytial virus infection is infant, child, or adult.

In preferred embodiments, the population having the human respiratory syncytial virus infection is infant aged 1 to 24 months, preferably infant aged 1 to 6 months.

In preferred embodiments, the sequelae of human respiratory syncytial virus infection are selected from the group consisting of cough, wheezing, asthma, shortness of breath, reactive airway disease, recurrent respiratory tract infections, and pulmonary function impairments.

In preferred embodiments, the sequelae of human respiratory syncytial virus infection are selected from the group consisting of wheezing and/or asthma.

In preferred embodiments, the method reduces at least one sequela selected from the group consisting of annual occurrence of wheezing, number of wheeze episodes, and probability of developing asthma.

In preferred embodiments, the method reduces the annualised rate of wheeze by a proportion of at least 30%, preferably at least 50%, and more preferably at least 70%. For example, the method reduces the annualised rate of wheeze by a proportion of 30%-80%, 35%-80%, 40%-80%, 50%-80%, 60%-80%, 70%-80%, 30%-75%, 35%-75%, 40%-75%, 45%-75%, 50%-75%, 30%, 35%, 40%, 45%, 50%, 55%, 60%, 65%, 70%, 75%, 72%.

In preferred embodiments, the method reduces the incidence of wheeze episodes by a proportion of at least 30%, preferably at least 40%, and more preferably at least 60%. For example, the method reduces the incidence of wheeze episodes by a proportion of 30%-80%, 35%-80%, 40%-80%, 50%-80%, 60%-80%, 30%-70%, 35%-70%, 40%-70%, 50%-70%, 60%-70%, 30%-65%, 35%-65%, 40%-65%, 50%-65%, 55%-65%, 30%, 35%, 40%, 45%, 50%, 55%, 60%, 61.5%.

In preferred embodiments, the method reduces the prevalence rate of asthma by a proportion of at least 30%, preferably at least 40%, and more preferably at least 50%. For example, the method reduces the prevalence rate of asthma by a proportion of 30%-60%, 35%-60%, 40%-60%, 50%-60%, 30%-55%, 35%-55%, 40%-55%, 45%-55%, 50%-55%, 30%, 35%, 40%, 45%, 50%, 51.3%.

As used herein, "and/or" means "either or both" of the elements so combined, i.e., elements that may be present conjointly in some cases and separately in other cases. Thus, by way of non-limiting example, when used in conjunction with open-ended language such as "comprising", "A and/or B" may, in some embodiments, refer only to A (optionally including elements other than B); in other embodiments, only to B (optionally including elements other than A); in other embodiments, to both A and B (optionally including other elements); and so on.

As used herein, when a numerical value is recited alone or as part of a numerical range, it should be understood that the numerical value can vary above and below the stated value with a variance that is reasonable for that value, as recognized by a person skilled in the art.

As used herein, the term "about" or "approximately" applied to one or more values of interest refers to a value similar to the reference value. In certain embodiments, the term refers to a range of values that fall within 10%, 9%, 8%, 7%, 6%, 5%, 4%, 3%, 2%, 1% or less in either direction (greater than or less than) of the reference value, unless otherwise stated or apparent from the context.

As used herein, the term "prevention" refers to preventing or at least reducing the likelihood of infection by at least one pathogenic microorganism in a human or animal organism. Under the effect of the ziresovir administration, it can effectively avoid or reduce the relevant clinical symptoms or indicators of the sequelae after the cure of RSV infection.

As used herein, the term "treatment" refers to combating the infection by at least one pathogenic microorganism in a human or animal organism. The administration of ziresovir would alleviate the symptoms associated with the infection (such as wheezing, asthma, etc.) and/or prevent/reduce the risk of complications, especially superinfections.

As used herein, the term " the effective amount " refers to the dosage standard of a certain substance (such as a drug, toxin, chemical reagent, radiation, etc.) administered to an organism (human or animal) or a biological system (such as cells, microorganisms) for a specific purpose (such as treatment, experiment, disinfection, etc.).

### Example 1

### 1.1 Preparation of ziresovir AK0529

Ziresovir is prepared according to the methods described in Examples 1-13 of International Application WO2015110446A1.

### 1.2 Preparations of AK0529 capsules and placebo capsules

The formulae of the AK0529 capsules and placebo capsules used in this disclosure are shown in the following table.

| Components | placebo 10 mg capsule (mg per capsule) | placebo 40 mg capsule (mg per capsule) | AK0529 10 mg capsule (mg per capsule) | AK0529 40 mg capsule (mg per capsule) | Function | Reference compendia |
|---|---|---|---|---|---|---|
| AK0529 | - | - | 10.00 | 40.00 | active ingredient | Internal control standard |
| Opadry | - | - | 4.37 | 17.49 | Isolation material | Internal control standard |
| Sugar Sphere | 95.53 | 382.1 | 78.62 | 314.47 | Drug carrier | United States Pharmacopeia/National Formulary, European and Chinese Pharmacopoeia |
| HPMC | - | - | 2.50 | 10.00 | Binder | United States, European, Japanese and Chinese Pharmacopoeia |
| EUDRAGIT L30D-55 | 12.24 | 48.97 | 12.24 | 48.97 | Enteric coating material | European Pharmacopoeia, National Formulary, Japanese Pharmacopoeia |
| Aqueous anti-sticking agent and plasticizer mixed emulsion PlasACRYL^{®} HTP 20 | 2.08 | 8.33 | 2.08 | 8.33 | Emulsifier, anti-sticking agent, plasticizer | Internal control standard |
| colloidal silicon dioxide | 0.33 | 1.32 | 0.33 | 1.32 | anti-sticking agent | European Pharmacopoeia, United States Pharmacopoeia/National Formulary, Japanese Pharmacopoeia |
| Talc | 0.12 | 0.46 | 0.11 | 0.44 | anti-sticking agent | European, United States and Japanese Pharmacopoeia |
| Total | 110.3 | 441.2 | 110.3 | 441.0 | | |
| Hypromellose hollow capsule | 1 capsule | 1 capsule | 1 capsule | 1 capsule | capsule shell | European, Japanese, and Chinese Pharmacopoeia, United States Pharmacopeia/National Formulary |

The preparation method of AK0529 capsules used in this disclosure includes:
1. Preparation of the isolation layer: Slowly add Opadry into an appropriate amount of water to form a suspension, and then perform the coating of the isolation layer.
2. Preparation of the drug layer: Disperse the AK0529 compound in an aqueous solution of hypromellose to form a suspension, and then perform the coating of the drug layer.
3. Preparation of the enteric coating layer: Add Eudragit L30D-55 and the mixed emulsion of the aqueous anti-sticking agent and plasticizer PlasACRYL^{®} HTP 20 into an appropriate amount of water to form a suspension, and then perform the coating of the enteric coating layer.
4. Preparation of the capsules: Add talcum powder and colloidal silicon dioxide into the coated pellets, mix carefully and process them into capsules.

The placebo capsules were prepared referring to the preparation method of AK0529 capsules.

### 1.3 Preclinical Studies of AK0529

### 1.3.1 Drug Resistance Mapping Test

To identify the molecular target of the inhibitor, drug resistant mapping studies were performed, and drug resistant mutants were selected in the presence of AK0529. Resistant viral isolates were obtained after several passages with increasing concentration of the compound. The RSV genome from the resistant isolates were amplified by reverse-transcription-PCR and sequenced in order to determine the viral protein that was targeted by the inhibitor. Each of the resistant isolates harbored a different single nucleotide substitution in the F gene, resulting in D486N(aspartic acid (Asp) 486 mutated to asparagine (Asn)), D489A(aspartic acid 489 mutated to alanine (Ala)), D489V(aspartic acid 489 mutated to valine (Val)), D489Y amino acid(aspartic acid 489 mutated to tyrosine (Tyr)) changes in passage 3, respectively. All the mutations rendered drug resistance to AK0529 were located in the hypervariable region of the C-terminal of RSV-F protein. The data indicated that the bona fide target of RO-0529 is RSV F protein.

### 1.3.2 Cell-cell Fusion Assay

The mode of action of AK0529 was further confirmed in an assay of inhibition of RSV F protein-induced cell-cell fusion process. RSV-F expression was induced by 1 µg/mL tetracycline in the RSV F protein stable cell line. AK0529 was serially diluted and added into the RSV-F expressing cells. The cell-cell fusion was observed by microscopy 3 days after AK0529 addition.

As shown in Figure 1, without RO-0529, RSV F protein induced cell fusion process and generated syncytia formation shown in the red dot cycles (left figure). In the presence of 0.1 µM AK0529, the syncytia formation induced by the RSV F protein was completely inhibited (right figure). These results further confirmed that AK0529 was a RSV F inhibitor, as there was no other RSV viral protein present except F protein.

### Example 2

This example describes the Phase III clinical trial (AIRFLO) of ziresovir (AK0529) against respiratory syncytial virus (RSV) infection in infant patients (AIRFLO ClinicalTrials.gov number: NCT04231968). This is a randomized, double-blind, placebo-controlled study to evaluate the efficacy, safety, and tolerability of AK0529 in hospitalized infants with RSV infection. The designed trial profile and the enrollment status of the subjects in this study are shown in Figure 2.

### 2.1 Participants

Participants were eligible if they were 1 to 24 months of age (with adjustment for prematurity), had a body weight of 2.5 to 20.0 kg, and were hospitalized with a diagnosis of RSV infection that was confirmed by virologic methods within 36 hours before receipt of the first dose of ziresovir or placebo in the trial. Participants also had to have a Wang bronchiolitis clinical score of at least 5 (on a scale from 0 to 12, with higher scores indicating greater severity of signs and symptoms) at the time of the first administration of ziresovir or placebo and had to have received the first dose within 7 days after the onset of symptoms of RSV infection.

### Exclusion criteria:

1.Patients who have used any prohibited medications (prohibited drugs include interferon, ribavirin, and Chinese patent medicines that are clearly listed as having antiviral effects in the drug instruction manuals, etc.) within 72 hours prior to expected administration and those who have used inhaled or systemic glucocorticosteroids within 24 hours prior to administration.
2.Patients (or mothers of patients younger than 6 months of age) with a known HIV-positive history or patients highly suspected HIV-positive by the investigator.
3. Patients with known co-infection with influenza virus.
4. Patient known to have pneumonia caused by bacterial infection.
5. Patients requiring vasopressors or vasoactive drugs at the time of enrollment.
6. Concurrent gastrointestinal conditions that could seriously, in the opinion of the investigator, prejudice absorption of the Investigational Medicinal Product.
7. Bronchopulmonary dysplasia requiring assisted ventilation at the time of enrolment, except for the result of RSV infection.
8. Patients at risk for hypercapnia based on their medical history, except for the result of RSV infection.
9. Patient with airway malformations and congenital heart diseases, except for isolated patent ductus arteriosus and/or patent foramen ovale.
10. Renal failure including renal abnormalities (clinical conditions such as abnormal renal development, polycystic kidney disease, renal hypoplasia) likely to be associated with renal insufficiency.
11. Clinical evidence of hepatic decompensation (such as liver diseases accompanied by coagulation abnormalities or brain lesions).
12. Symptomatic because of congenital metabolic abnormality (such as mitochondrial diseases, abnormal carbohydrate metabolism, glycogen storage diseases).
13. Chronic or persistent feeding difficulties.
14. Known or suspected to have primary immunodeficiency disease (such as congenital agammaglobulinemia).
15. Any active or uncontrolled respiratory, cardiac, hepatic, central nervous system or renal disease unrelated to RSV infection at baseline, or any other medical condition that in the opinion of the investigator renders the patient unsuitable for enrolment; in case of any question, discuss such cases with the sponsor's medical monitor.
16. A history of epilepsy or seizures including febrile seizures.
17. History of family history of high allergies or allergies to multiple substances, or presence of severe rash that in the opinion or the investigator renders the patient unsuitable for enrollment.
18. The patient's parent or guardian is an employee of the investigator or the study site with direct involvement in the proposed study or other studies under the direction of that investigator of the study site, or any family members of the employees or the investigator.
19. Participation in an investigational drug or device study within 30 days prior to the date of screening.
20. Failure to satisfy the investigator of fitness to participate for any other reason.

### 2.2 Trial Design and Oversight

This double-blind, randomized, placebo-controlled trial in two parts were conducted at 30 clinical sites at 28 hospitals in China. Part 1 was a safety and pharmacokinetic analysis, and part 2 evaluated efficacy. Details of the trial design are shown in Figure 1.

### 2.3 Randomization

Participant randomization tables were generated by independent statisticians using the Medidata Randomization and Trial Supply Management (PTSM) system. In both parts of the trial, participants were randomly assigned, in a 2:1 ratio, to receive ziresovir or placebo. Block randomization was performed (blocks of 20 in part 1 and 100 in part 2) without stratification.

The grouping and dosing regimen in the trial are as follows:
Ziresovir group 1: participants weighing≥ 2.5 kg to < 5.0 kg: ziresovir capsules (10 mg per capsule), 1 capsule each time in oral, twice a day, successive administration for 5 days.
Ziresovir group 2: participants weighin≥ 5.0 kg to < 10.0 kg: ziresovir capsules (10 mg per capsule), 2 capsules each time in oral, twice a day, successive administration for 5 days.
Ziresovir group 3: participants weighing≥ 10.0 kg to < 20.0 kg: ziresovir capsules (40 mg per capsule), 1 capsule each time in oral, twice a day, successive administration for 5 days.
Placebo group 1: participants weighin≥ 2.5 kg to < 5.0 kg: Placebo capsules (10 mg per capsule), 1 capsule each time in oral, twice a day, successive administration for 5 days.
Placebo group 2: participants weighin≥ 5.0 kg to < 10.0 kg: Placebo capsules (10 mg per capsule), 2 capsules each time in oral, twice a day, successive administration for 5 days.
Placebo group 3: participants weighing≥ 10.0 kg to < 20.0 kg: Placebo capsules (40 mg per capsule), 1 capsule each time in oral, twice a day, successive administration for 5 days.

Ziresovir or placebo was administered every 12 hours (within a window of ±2 hours) relative to the first dose on day 1 for 5 days. An independent data and safety monitoring board reviewed the safety data and determined whether ziresovir or placebo should be discontinued.

Part 2 of the trial was initiated after part 1 was completed. Participants received ziresovir or placebo for 5 days, with a 9-day safety follow-up period. Protocol amendments included extension to a 6-month follow-up period and further to a 2-year follow-up to assess the frequency, duration, and treatment of parent-reported wheeze and the frequency of asthma diagnosis. Parent-reported wheeze was the first episode of wheezing observed by parents following each patient's discharge to home. Parents were required to record each episode of subsequent wheezing. Data for the follow-up period were collected through multiple telephone inquiries conducted by nurses and physicians over a period of 2 years, recorded in a standard form and evaluated collectively at 6-month intervals.

### 2.4 End Points and Assessments

Part 1 of the trial included no efficacy end points. Participants' safety data were reviewed by the data and safety monitoring board to determine whether recruitment should continue. Safety assessment was performed three times, after the recruitment of 6, 18, and 54 participants.

Efficacy was assessed in part 2. The primary efficacy end point was the change from baseline to day 3 (i.e., 48 hours after the first administration of ziresovir or placebo) in the Wang bronchiolitis clinical score (bronchiolitis score). This instrument assesses the severity of signs and symptoms related to lower respiratory tract infection, including the respiratory rate(0 to 3 scores), wheezing (0 to 3 scores), retraction of respiratory muscles (0 to 3 scores), and general condition (0 to 3 scores); each sub-score is assessed on a scale from 0 (normal) to 3 (most severe), with the total score ranging from 0 to 12. The standard for each sub-score is as follows:
Respiratory rate: 0 score (Breathe per minute≤30 times); 1 score (Breathe per minute from 31 to 45 times); 2 score (Breathe per minute from 46 to 60 times); 3 score (Breathe per minute>60 times)
Wheezing: 0 score indicate no wheezing; 1 score indicate that wheezing can only be heard at the end of exhalation or with the aid of a stethoscope; 2 score indicate that wheezing can be heard throughout the exhalation process or without the need for a stethoscope; 3 score indicate that wheezing can be heard during both inhalation and exhalation without the need for a stethoscope, or no wheezing sound can be heard due to extremely little air intake.
Retraction of respiratory muscles: 0 score (no muscle retraction); 1 score (retraction only in the intercostal muscles); 2 score (visible retraction of the jugular notch); 3 score (accompanied by severe signs such as nasal flaring).
General condition: 0 score indicates normal; 3 score indicates an irritable or lethargic condition or poor feeding.

Each participant's score was evaluated by the same investigator when possible and completed within 1 hour on a similar schedule before the first dose of the day was administered from day 1 to day 6 or until early withdrawal.

The key secondary end point was the change in the RSV viral load from baseline to day 5 (i.e., 96 hours after the first administration of ziresovir or placebo). Viral specimens from the same participant were obtained from the same anatomical location by means of nasopharyngeal aspiration within 1 hour before the first dose each day and on days 6 and 14 or once before early withdrawal. The viral load was determined by means of a TaqMan quantitative reverse-transcriptase-polymerase-chain-reaction (RT-PCR) assay at Wuxi AppTec. The proportions of participants who had a viral load below the lower limit of quantification (LLOQ) were calculated.

Additional secondary end points included the change from baseline to day 3 in the bronchiolitis score among participants with a baseline score of 8 or higher, the change from baseline to day 3 in the bronchiolitis score among participants 6 months of age or younger, symptom remission (defined as a bronchiolitis score of 0 or 1) on day 3, a reduction of at least 75% from baseline in the bronchiolitis score on day 3, the time to the first symptom remission after the first dose of ziresovir or placebo was administered, the time to a bronchiolitis general condition sub-score of 0 among participants with general symptoms at baseline, and the median time to a reduction of at least 75% from baseline in the bronchiolitis score. Among participants who had a stay in the intensive care unit (ICU), the duration of stay was assessed. Participants were discharged from the hospital when clinical symptoms and signs had abated (i.e., if wheezing had ceased, cough had abated, and fever had subsided for >3 days). Sequencing of the RSV F gene was performed to monitor the appearance of drug-resistant mutations, and detection of coinfection with other respiratory viruses was conducted.

Adverse events and serious adverse events were monitored throughout the trial and coded with the use of the Medical Dictionary for Regulatory Activities, version 24.1. Safety signals were evaluated by the data and safety monitoring board.

### 2.5 Statistical Analysis

In part 2 of the trial, the efficacy analyses were conducted in the intention-to-treat population, which included all the participants who had RT-PCR-confirmed RSV infection and had received at least one dose of ziresovir or placebo after randomization. Safety data were analyzed in the safety population, which included all the participants who had received ziresovir or placebo at least once after randomization in part 1 or part 2.

The primary efficacy end point was analyzed with the use of a mixed model for repeated measures. The model included the change from baseline (day 1) in the Wang bronchiolitis clinical score from day 2 to day 6 as the dependent variable, with the assessment time point, trial group, and assessment time point-trial group interaction as fixed effects. The model was adjusted for baseline covariates, including the baseline bronchiolitis score, age (≤6 months or >6 to 24 months), and time to onset of RSV infection. The RSV viral load was analyzed with a repeated measures model of restricted maximum-likelihood estimation. Details of the models we used, along with methods of other statistical analyses, are provided in the conventional methods in this field.

In part 2, the primary efficacy end point and the key secondary efficacy end point were tested for superiority at a two-sided level of 0.05 with the use of the hierarchical fixed-sequence method. Subgroup analyses (in patients aged ≤6 months) of the primary endpoint and important secondary endpoints (ie, RSV viral load change, 75% reduction in WBCS from baseline, and proportion of patients with disease remission) were prespecified. Additional analyses presented in this disclosure were post hoc, and p values should be considered nominal. The proportion of patients with a 75% reduction in WBCS, proportion with remission, time to first hospital discharge from the first dose during the study period, and time to first cessation of supplemental oxygen from the first dose during study period were analyzed using both Cochran-Mantel-Haenszel and logistic regression. Time-to-event variables (including parent-reported wheeze over 2 years of follow-up) were compared with Kaplan-Meier analysis, stratified log-rank test (with stratification by the potential confounders of age [≤6 months or >6 months) and time since RSV onset (≤3 days or >3 days]), and Cox proportional hazards regression models. The cumulative probability of parent-reported wheeze during the 2-year follow-up was analyzed, and a negative binomial regression model was used to analyze the annualized rate of parent-reported wheeze. Viral load and WBCS at each timepoint were compared using t tests. In post-hoc analyses, the correlations between RSV viral load and WBCS (absolute values at baseline; and changes from baseline at days 3 and 5), and between oxygen saturation (SpO2) and WBCS (absolute values at baseline, day 3, and day 5; and changes from baseline at days 3 and 5) were evaluated by Spearman's rank correlation coefficients. All the statistical analyses were performed with the use of SAS software, version 9.4 (SAS Institute).

### 2.6 Results of the Phase III clinical trial

### 2.6.1 Trial Population

As shown in Figure 2, of the 355 patients aged 1-24 months who were screened, 311 were enrolled and randomly allocated across parts 1 and 2 of the study: 205 (66%) to the ziresovir group and 106 (34%) to the placebo group. 302 participants received at least one dose of study drug, among whom 188 were aged 6 months or younger (125 in the ziresovir group and 63 in the placebo group) and were analyzed for safety in this study. Per protocol, part 1 of the study was for safety and pharmacokinetic assessment. 150 patients in part 2 of the study (100 in the ziresovir group and 50 in the placebo group) comprised the ITT-i population of patients aged 6 months or younger, which was analyzed for efficacy. In the ziresovir group, 33 (26%) of 125 patients were female and 92 (74%) were male. In the placebo group, 15 (24%) of 63 patients were female and 48 (76%) were male. Baseline mean WBCS was 6.8 (SD 1.7) in the ziresovir group and 6.9 (1.8) in the placebo group. 63 (50%) patients in the ziresovir group and 26 (41%) in the placebo group needed oxygen supply or non-invasive positive pressure ventilation at baseline. Baseline demographic and disease characteristics of the ITT-i population are shown in the appendix (p 4). Of the ITT-i population, 96 (96%) of 100 patients in the ziresovir group and 47 (94%) of 50 in the placebo group completed the study. Three (3%) patients from the ziresovir group discontinued the study due to adverse events (one instance each of thrombocytopenia, anomalous pulmonary venous connection, and rash), while no patients discontinued placebo due to adverse events.

### 2.6.2 Pharmacokinetics

Results of the pharmacokinetic-pharmacodynamic correlation analysis suggested that a trough drug concentration (Ctrough) of at least 130 ng per milliliter was associated with greater decreases (indicating improvements) in clinical symptom scores and a greater reduction in the viral load and that a minimum dose of 2 mg per kilogram of body weight was required in order to reach a Ctrough of 130 ng per milliliter in more than 80% of the participants.

At 12 hours after administration of the drug, the mean Ctrough of ziresovir at steady state in blood was 378±217 ng per milliliter, which exceeded the target concentration of 130 ng per milliliter.

### 2.6.3 Efficacy

Treatment duration ranged from 0.5 to 5.1 days in each group, with a mean 4.9 days (SD 0.7) and median 5.0 days in the ziresovir group and a mean 4.8 days and median 5.0 days (4·9 to 5·0) in the placebo group. At day 3 (48 h after baseline), patients 6 months and younger who received ziresovir showed significant improvement in the primary endpoint of change from baseline in WBCS (least-squares mean -3.5 points) compared with patients who received placebo (-2.2 points), with a between-group difference of-1.2 points (-1.9 to -0.6; p=0.0004) or an additional 54.5% reduction with ziresovir than with placebo( as seen in Figure 3A). Treatment with ziresovir also resulted in statistically greater reductions in WBCS component scores compared with placebo from baseline to day 3(as seen in Figure 3B). Mean WBCS differed significantly between the ziresovir and placebo groups on day 3 (3.4 vs 4·7; p=0·0002), day 4, and day 5.

Reductions in viral load during treatment (the key secondary endpoint) were greater with ziresovir (-2.51 log10 copies per mL [95% CI -2.87 to -2.14]) than with placebo (-1.87 log10 copies per mL [-2.35 to -1.38]) at day 5 (96 h after baseline), a difference of -0.64 log10copies per mL (-1.20 to -0.09; p=0.024), or a 77% reduction compared with placebo. This difference in viral load reduction with ziresovir was also observed at day 3 (-0.45 log10 copies per mL; p=0.049; figure 4A). At day 5, 27 (29% [95% CI 20-39]) of 93 patients who received ziresovir had a viral load under the LLOQ, compared with seven (15% [6-29]) of 46 who received placebo (Figure 4B). A significant difference between the ziresovir and placebo groups in mean RSV viral load was seen at day 5 (5.0 log10 copies/mL [95% CI 4.6-5.4] vs 5.7 log10 copies/mL [5.3-6.1]; p=0.011).

The above results indicate that treatment with ziresovir leads to significant clinical improvement and greater viral load reduction compared to placebo. The antiviral effect of ziresovir was observed as early as day 3. The delayed effect of viral load reduction with ziresovir is consistent with the mechanism of action of F protein inhibitors, which can block viral entry into cells but have no direct effect on intracellular viruses.

### 2.6.4 Follow-up of patients

The 24-month post-discharge follow-up data, from a mean 15.33 months (SD 9.44) for ziresovir and 17.14 months (9.02) for placebo, were available for 116 infants who had been aged 6 months or younger and hospitalized for RSV infection at study entry (78 who received ziresovir and 38 who received placebo).

The hazard ratio for time to first parent-reported wheeze recurrence was 0.41 between the ziresovir and placebo groups (Figure 5). The mean annualised rates of parent-reported wheeze were 0.18 episodes in the ziresovir group and 0.65 episodes in the placebo group (p=0.0048)-3.6 times lower in the ziresovir group versus the placebo group(The mean annualised rate of wheeze in the ziresovir group was 27.78% of that in the placebo group.).That is, the mean annualised rate of wheeze in the ziresovir group decreased by 72.3% compared to that in the placebo group. Among those aged 6 months or younger at randomization who had parent-reported wheeze during follow-up, there were a mean 1.2 wheeze episodes (SD 0·6) in the ziresovir group and 3.1 wheeze episodes (4·4) in the placebo group during 2-year follow-up per patient-2.6 times lower in the ziresovir group versus the placebo group(The incidence of wheeze episodes in the ziresovir group is 38.7% of that in the placebo group). That is, the incidence of wheeze episodes in the ziresovir group decreased by 61.5% compared to that in the placebo group. One patient in the ziresovir group and two patients in the placebo group had three or more episodes of wheezing over the course of a year. A smaller proportion of patients were diagnosed with asthma during follow-up in the ziresovir group (two [2.56%] of 78) compared with the placebo group (two [5.26%] of 38) (the asthma prevalence rate in the ziresovir group is 48.7% of that in the placebo group.). That is, the prevalence rate of asthma in the ziresovir group decreased by 51.3% compared to that in the placebo group.

These data indicates that treatment with ziresovir can reduce the annual number of parent-reported wheeze, the wheeze episodes, and the likelihood of developing asthma in patients aged 6 months or younger, providing significant long-term benefits to infants and young children infected with RSV.

It should be understood that the above examples are all exemplary and are not intended to cover all possible embodiments encompassed by the claims. Various modifications and changes can still be made on the basis of the above examples without departing from the scope of the present disclosure. Similarly, the various technical features of the above examples can also be arbitrarily combined to form additional examples that may not have been explicitly described herein. Therefore, the above examples only express several embodiments of the present invention and do not limit the protection scope of the present invention.

## Claims

1. Ziresovir or its pharmaceutically acceptable salt for use in the preparation of a drug for preventing or treating sequelae of human respiratory syncytial virus infection, wherein an effective amount of the ziresovir or its pharmaceutically acceptable salt is administered to a subject in need thereof.

2. The ziresovir or its pharmaceutically acceptable salt for use according to claim 1, wherein the effective amount is 1~20 mg/kg/d, preferably 2~15 mg/kg/d, more preferably 4~12 mg/kg/d.

3. The ziresovir or its pharmaceutically acceptable salt for use according to claim 1 or 2, wherein the ziresovir or its pharmaceutically acceptable salt is administered during the treatment period of the acute infection.

4. The ziresovir or its pharmaceutically acceptable salt for use according to any one of claims 1 to 3, wherein the ziresovir or its pharmaceutically acceptable salt is administered twice a day.

5. The ziresovir or its pharmaceutically acceptable salt for use according to any one of claims 1 to 4, wherein the ziresovir or its pharmaceutically acceptable salt is administered orally for 5 consecutive days.

6. The ziresovir or its pharmaceutically acceptable salt for use according to any one of claims 1 to 5, wherein a population having the human respiratory syncytial virus infection is infant, child, or adult.

7. The ziresovir or its pharmaceutically acceptable salt for use according to any one of claims 1 to 6, wherein a population having the human respiratory syncytial virus infection is infant aged 1 to 24 months, preferably infant aged 1 to 6 months.

8. The ziresovir or its pharmaceutically acceptable salt for use according to any one of claims 1 to 7, wherein the sequelae of human respiratory syncytial virus infection are selected from the group consisting of cough, wheezing, asthma, shortness of breath, reactive airway disease, recurrent respiratory tract infections, and pulmonary function impairments.

9. The ziresovir or its pharmaceutically acceptable salt for use according to claim 8, wherein the sequelae of human respiratory syncytial virus infection are selected from the group consisting of wheezing and/or asthma.

10. The ziresovir or its pharmaceutically acceptable salt for use according to any one of claims 1 to 9, wherein the use reduces at least one sequela selected from the group consisting of annual occurrence of wheezing, number of wheeze episodes, and probability of developing asthma.

11. The ziresovir or its pharmaceutically acceptable salt for use according to any one of claims 1 to 10, wherein the use reduces an annualized rate of wheeze by a proportion of at least 30%, preferably at least 50%, and more preferably at least 70%.

12. The ziresovir or its pharmaceutically acceptable salt for use according to any one of claims 1 to 10, wherein the use reduces an incidence of wheeze episodes by a proportion of at least 30%, preferably at least 40%, and more preferably at least 60%.

13. The ziresovir or its pharmaceutically acceptable salt for use according to any one of claims 1 to 10, wherein the use reduces the prevalence rate of asthma by a proportion of at least 30%, preferably at least 40%, and more preferably at least 50%.

14. A pharmaceutical composition for use in the preparation of a drug for preventing or treating sequelae of human respiratory syncytial virus infection, wherein the pharmaceutical composition comprises ziresovir and at least one pharmaceutically acceptable ingredient.

15. The ziresovir or its pharmaceutically acceptable salt for use according to any one of claims 1-13 or the pharmaceutical composition for use according to claim 14, wherein the drug or the pharmaceutical composition is powder, pill, tablet, capsule, oral liquid, aerosol or injection, preferably capsule.
